(19) Europäisches Patentamt
European Patent Office
Office européen des brevets

(11) Numéro de publication: **0 362 048 A1**

(12) # DEMANDE DE BREVET EUROPEEN

(21) Numéro de dépôt: **89402628.5**

(22) Date de dépôt: **26.09.89**

(51) Int. Cl.5: **A61K 7/06 , A61K 7/48 , C08G 77/38**

Revendications pour les Etats contractants suivants: ES + GR.

(30) Priorité: **28.09.88 LU 87350**

(43) Date de publication de la demande: **04.04.90 Bulletin 90/14**

(84) Etats contractants désignés: **AT BE CH DE ES FR GB GR IT LI NL SE**

(71) Demandeur: **L'OREAL**
**14, Rue Royale**
**F-75008 Paris(FR)**

(72) Inventeur: **Grollier, Jean-François**
**16bis Boulevard Morland**
**F-75004 Paris(FR)**
Inventeur: **Dubief, Claude**
**9, Rue Edmond Rostand**
**F-78150 Le Chesnay(FR)**

(74) Mandataire: **Casalonga, Axel et al**
**BUREAU D.A. CASALONGA - JOSSE**
**Morassistrasse 8**
**D-8000 München 5(DE)**

(54) **Application cosmétique et dermatologique de polysiloxanes à fonction diester et compositions mises en oeuvre.**

(57) L'invention concerne l'utilisation en cosmétique et en dermatologie d'au moins un diorganopolysiloxane à fonction diester portant, par molécule, au moins un motif de formule :

$$ZR_aSiO_{\frac{3-a}{2}}$$

où a est 0,1 ou 2, Z est un radical de formule :

$$W-\underset{X}{\overset{COOR'}{C}}-CH_2-COOR'$$

les symboles R′, identiques ou différents, désignent un radical hydrocarboné saturé, monovalent en $C_1$-$C_{12}$, alcoxyalkyle en $C_2$-$C_{12}$, aryle, alkylaryle ou aralkyle en $C_6$-$C_{12}$; X désigne H ou $CH_3$; W est une liaison covalente ou un radical alkylène en $C_1$-$C_4$; les symboles R, identiques ou différents, désignent alkyle en $C_1$-$C_{20}$, vinyle, phényle, trifluoro-3,3,3 propyle ou hydroxyle, sous réserve qu'un seul radical R par atome de silicium désigne hydroxyle.



EP 0 362 048 A1

# Application cosmétique et dermatologique de polysiloxanes à fonction diester et compositions mises en oeuvre.

La présente invention a pour objet l'utilisation cosmétique et dermatologique de diorganopolysiloxanes à fonction diester, aux compositions cosmétiques et dermatologiques mises en oeuvre ainsi qu'au procédé de traitement cosmétique, en particulier des cheveux et de la peau.

On utilise déjà des huiles siliconées en cosmétique comme lubrifiant dans les compositions destinées au traitement et aux soins des cheveux et de la peau. Il s'agit essentiellement de polydiméthylsiloxanes.

La demanderesse a découvert, de façon surprenante, que l'utilisation d'huiles siliconées constituées de diorganopolysiloxanes à fonction diester permettait d'obtenir des cheveux brillants, doux, se démêlant aisément tout en ayant un toucher non gras. Ces composés présentent également l'avantage de conférer à la peau de la douceur et un toucher non poisseux.

On appelle "traitement cosmétique" un traitement visant à obtenir sur les cheveux un ou plusieurs des résultats indiqués ci-dessus. Il en est de même pour le traitement cosmétique de la peau.

Un objet de l'invention est donc constitué par l'utilisation cosmétique et dermatologique des diorganopolysiloxanes à fonction diester.

L'invention a également pour objet un procédé de traitement cosmétique des cheveux ou de la peau mettant en oeuvre ces composés.

Un autre objet de l'invention est constitué par les compositions cosmétiques et dermatologiques destinées au traitement de la peau ou des cheveux, mettant en oeuvre ces composés.

D'autres objets de l'invention apparaîtront à la lecture de la description et des exemples qui suivent.

La présente invention a donc pour objet principal l'utilisation cosmétique et dermatologique de diorganopolysiloxanes à fonction diester, portant par molécule au moins un motif de formule :

$$ZR_aSiO_{\frac{3-a}{2}} \qquad (I)$$

dans laquelle :

- a est 0, 1 ou 2
- Z est choisi parmi les radicaux de formule :

$$W-\underset{\underset{X}{|}}{\overset{\overset{COOR'}{|}}{C}}-CH_2-COOR'$$

dans laquelle :

les symboles R′, identiques ou différents, désignent un radical hydrocarboné saturé, monovalent en $C_1$-$C_{12}$, un radical alcoxyalkyle monovalent en $C_2$-$C_{12}$, un radical aryle, alkylaryle ou aralkyle en $C_6$-$C_{12}$;

X désigne un atome d'hydrogène ou un radical méthyle;

W désigne une liaison covalente ou un radical alkylène, linéaire ou ramifié en $C_1$-$C_4$;

les symboles R, identiques ou différents, désignent un radical alkyle en $C_1$-$C_{20}$, vinyle, phényle, trifluoro-3,3,3 propyle ou hydroxyle, sous réserve qu'un seul des radicaux R par atome de silicium puisse être un hydroxyle;

ou un mélange de ces composés.

Les autres motifs siloxyle de l'organopolysiloxane répondent à la formule :

$$R_bSiO_{\frac{4-b}{2}} \qquad (Ibis)$$

dans laquelle :

- R a la même signification que ci-dessus; et
- b est égal à 0, 1, 2 ou 3.

Les groupements W alkylènes préférés, lorsque X désigne un atome d'hydrogène, sont choisis parmi les radicaux suivants :

$-CH_2-$

$-(CH_2)_3-$

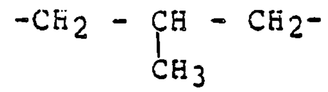

X désigne de préférence un groupement méthyle lorsque le groupement W est une simple liaison covalente.

R′ désigne de préférence un radical alkyle en $C_1$-$C_{12}$ choisi en particulier parmi les radicaux méthyle, éthyle, n-propyle, isopropyle, n-butyle, éthyl-2 hexyle, heptyle et dodécyle.

Les radicaux aryle, arylalkyle et alkylaryle en $C_6$-$C_{12}$ préférés, représentés par R′, sont choisis parmi les radicaux phényle, benzyle et tolyle.

Les radicaux alcoxyalkyle en $C_2$-$C_{12}$ préférés, représentés par R′, selon l'invention, sont les groupements méthoxyméthyle, éthoxyméthyle et méthoxy-2 éthyle.

Les radicaux R alkyle préférés, conformes à l'invention, sont méthyle, éthyle, propyle, n-butyle, n-octyle et éthyl-2 hexyle.

De préférence, 80% en nombre au moins des radicaux R dans les composés conformes à l'invention sont méthyle.

Les composés plus particulièrement préférés, selon l'invention, sont les polymères cycliques ou linéaires, choisis parmi :

(i) les polysiloxanes répondant à la formule :

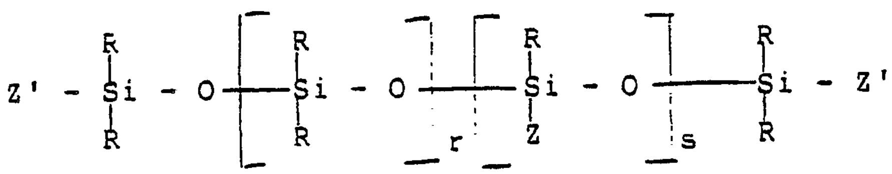 (II)

dans laquelle :

R a la même signification que celle donnée ci-dessus;

les symboles Z′, identiques ou différents, sont choisis parmi R et Z;

Z a la même signification que celle indiquée ci-dessus et est choisi, de préférence, parmi les radicaux

$-CH_2CH(COOR')CH_2COOR'$

$-C(CH_3)(COOR')CH_2COOR'$

R′ ayant les mêmes significations définies ci-dessus;

r est un nombre entier compris entre 0 et 500 inclusivement;

s est un nombre entier choisi entre 0 et 50 inclusivement; et

si s est 0, au moins un des deux symboles Z′ est Z;

(ii) les polysiloxanes cycliques de formule :

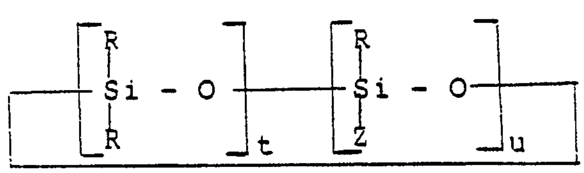 (III)

dans laquelle :

R et Z ont la même signification que ci-dessus;

u est un nombre entier compris entre 1 et 20 inclus; et

t est un nombre entier compris entre 0 et 20 inclus;

t + u est supérieur ou égal à 3.

On préfère plus particulièrement les polymères statistiques ou à blocs de formules (I), (II) et (III), présentant au moins l'une des caractéristiques suivantes :

R et R′ sont méthyle
r est compris entre 5 et 50 inclus
s est compris entre 1 et 20 inclus
t + u est compris entre 3 et 10 inclus.

Les polymères selon l'invention peuvent être préparés, notamment, selon un premier procédé (A).

Au cours d'une première étape ($A_1$), on additionne un diester organique de formule :

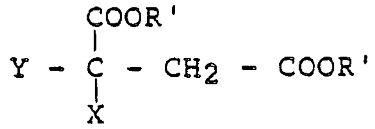
(IV)

ou de formule :

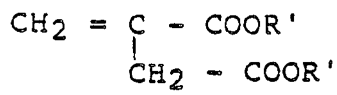
(IVbis)

Y étant choisi parmi un groupe alcényle, linéaire ou ramifié, ayant de 2 à 4 atomes de carbone inclus;
R′ et X ayant la même signification qu'à la formule (I),
sur un hydrogénoorganosilane de formule :
$H{-}R_a{-}Si{-}Cl_{3-a}$ (V)
R et a ayant la même signification qu'à la formule (I) ci-dessus.

On obtient ainsi un produit d'addition de formule :
$Z{-}R_a{-}Si{-}Cl_{3-a}$ (VI)
dans laquelle R, Z et a ont la même signification qu'à la formule (I) ci-dessus.

L'étape ($A_1$) peut être effectuée en masse ou en solution dans un solvant organique.

La réaction est exothermique. On opère généralement sous reflux du mélange réactionnel à une température comprise entre 60 et 140° C pendant une durée comprise entre 10 minutes et 3 heures.

On peut, soit couler le silane de formule (V) sur le diester de formule (IV) et/ou (IV bis) ou vice-versa, ou en même temps.

Il est préférable d'utiliser un excès molaire (de 10 à 50%) de silane de formule (V).

Il est préférable en vue d'augmenter la cinétique de réaction, d'opérer en présence d'un catalyseur. Les catalyseurs utilisables sont ceux utilisés pour faire la réaction d'hydrosilylation; sont donc en particulier utilisables, les peroxydes organiques, les radiations UV et les catalyseurs à base d'un métal du groupe du platine, en particulier le platine, le rhuténium et le rhodium à la dose de 10 à 500 ppm (calculés en poids de métal) par rapport au poids de silane de formule (V).

Comme exemples de catalyseurs, on peut citer le platine métal sur noir de carbone, les complexes platine/oléfine décrits dans les brevets américains US-A-3.159.601 et US-A-3.159.662, l'acide chloroplatinique, l'acide chloroplatineux, les complexes du platine et d'un vinylpolysiloxane décrits dans le brevet américain US-A-3.419.593, les complexes du platine de degré voisin de zéro, décrits dans les brevets américains US-A-3.715.334, US-A-3.775.452, US-A-3.814.730 et les complexes de platine avec un produit organique à insaturation éthylénique, décrits dans les brevets européens EP-A-188.978 et EP-A-190.530.

En fin de réaction, les produits volatils sont éliminés par distillation sous vide. Le vide de la pompe à eau de 0,1 à 3 KPa est généralement suffisant.

Au cours d'une deuxième étape ($A_2$), on effectue l'hydrolyse (ou la cohydrolyse) et la polycondensation d'un silane de formule (VI).

Cette hydrolyse ou cohydrolyse et polycondensation peuvent être effectuées de préférence en phase aqueuse liquide en milieu acide (de préférence HCl) ou en milieu basique (de préférence $NH_4OH$) ou en milieu solvant, dans des conditions similaires à celles de l'hydrolyse des chlorosilanes telles que décrites aux pages 193 à 200 de l'ouvrage de NOLL "CHEMISTRY and TECHNOLOGY of SILICONES" Academic Press (1968).

La concentration en acide ou en base dans l'eau est comprise généralement entre 10 et 30% en poids. Le milieu d'hydrolyse comporte toujours au moins 2 moles d'eau par mole de silane, généralement de 10 à 100 moles d'eau. L'hydrolyse peut être effectuée en continu ou en discontinu à température ambiante (20° C) ou à une température comprise entre 5 et 90° C. L'hydrolyse peut être effectuée à pression égale ou supérieure à la pression atmosphérique en continu ou en discontinu avec, au moins pour le procédé continu, réinjection d'eau pour maintenir une phase aqueuse constante.

En vue d'obtenir les polymères de formules (II) et (III) ou leurs mélanges, on hydrolyse et polycondense

les silanes de formule (VI) dans laquelle a = 1 en présence éventuellement d'un dichlorodiorganosilane de formule :

$R_2SiCl_2$ (VII)

dans laquelle R a la définition donnée à la formule (I) ci-dessus.

La polycondensation peut être arrêtée par simple neutralisation du milieu réactionnel. Dans ce cas, les polymères de formule (II) obtenus sont bloqués à chacune de leurs extrémités par un groupe hydroxyle (silanol) ou par le motif $R_2ZSiO_{0,5}$ si on utilise le silane $R_2ZSiCl$.

On peut également arrêter la polycondensation en ajoutant un composé organosilicié susceptible de réagir avec les hydroxyle terminaux, tels que les produits de formule :

$R_3$ SiCl; $R_3$ SiNHSi $R_3$ et $R_3$ $SiOSiR_3$

dans lesquelles les radicaux R ont la signification donnée à la formule (I) ci-dessus.

La durée de l'hydrolyse peut être comprise entre quelques secondes et plusieurs heures.

Après hydrolyse, la phase aqueuse est séparée de la phase siloxane par tout moyen physique approprié, généralement par décantation et extraction par un solvant organique comme l'éther isopropylique.

La phase siloxane peut être ultérieurement lavée à l'eau, puis distillée éventuellement pour séparer les polymères linéaires de formule (II) des polymères cycliques de formule (III).

Pour préparer les polymères de formules (I), (II) et (III), on peut également selon un deuxième procédé (B), partir du polymère correspondant dans lequel tous les radicaux Z et éventuellement Z′, sont des atomes d'hydrogène et par une réaction d'hydrosilylation, additionner un diester de formules (IV) et (IVbis) ci-dessus.

Ce polymère est dénommé par la suite polymère à SiH; les groupes SiH peuvent être présents dans la chaîne et/ou aux extrémités de chaîne. Ces polymères à SiH sont des produits bien connus dans l'industrie des silicones et sont généralement disponibles dans le commerce.

Ils sont par exemple décrits dans les brevets américains US-A-3.220.972, US-A-3.436.366, US-A-3.697.473 et US-A-4.340.709.

Ce polymère à SiH peut donc être représenté par la formule :

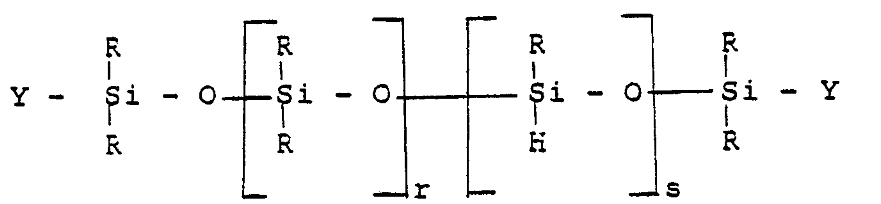

(VIII)

dans laquelle R, r et s ont la signification donnée ci-dessus pour la formule (II) et les radicaux Y, identiques ou différents, sont choisis parmi les radicaux R et un atome d'hydrogène;

et la formule :

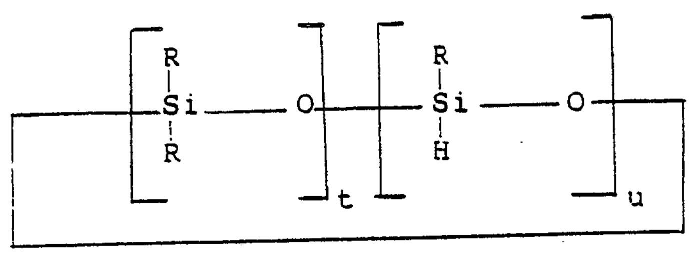

(IX)

dans laquelle R, t et u ont la signification donnée ci-dessus pour la formule (III).

Le procédé (B) met donc en oeuvre comme à l'étape ($A_1$) du procédé (A), une réaction d'hydrosilylation analogue et il est souhaitable d'effectuer cette réaction avec les mêmes catalyseurs que ceux indiqués à l'étape ($A_1$).

Cette réaction peut s'effectuer en masse ou au sein d'un solvant organique à une température comprise entre la température ordinaire (25 °C) et 170 °C.

Les produits volatils sont éliminés en fin de réaction par distillation sous vide et/ou par extraction.

Le procédé (A) permet d'obtenir des polymères de formule (II) présentant des groupes hydroxyle terminaux et des polymères de formules (II) et (III) présentant des radicaux R dont certains peuvent être

des radicaux vinyle.

Le procédé (B) permet d'avoir des polymères de structure bien définie par le choix des polymères à SiH de départ.

Comme produit de formule (IV), on préfère plus spécialement utiliser :

les diesters de l'acide allylsuccinique ou méthallylsuccinique, éventuellement α-méthylé (formule (IV)), dans laquelle :

Y est $CH_2=CH-CH_2-$ ou $CH_2=C(CH_3)-CH_2-$

et X est H ou $CH_3$).

Les organopolysiloxanes modifiés obtenus par les procédés (A) et (B) ci-dessus, sont également décrits dans les brevets américains US-A-4.207.246, US-A-4.322.473 et US-A-4.405.469, cités comme références.

- les diesters de l'acide itaconique (formule IV bis); on obtient alors deux formes isomères de radicaux Z :

- $CH_2CH(COOR')CH_2COOR'$ (formule I : W est $-CH_2-$, X est H); et

- $C(CH_3)(COOR')CH_2COOR'$ (formule I : W est une liaison de valence et X est méthyle).

Les diorganopolysiloxanes à fonction diester utilisés conformément à l'invention, se présentent généralement sous forme d'huiles plus ou moins visqueuses, de viscosité comprise entre 2 et 500.000 m.Pa.s, de préférence entre 5 et 5.000 m.Pa.s à 25° C.

Un autre objet de l'invention est constitué par les compositions cosmétiques pour le traitement et le soin des cheveux et de la peau, contenant dans un milieu cosmétiquement acceptable, un diorganopolysiloxane à fonction diester renfermant par molécule au moins un motif de formule (I), dans des concentrations comprises entre 0,2 et 90% et de préférence entre 0,3 et 60% en poids par rapport au poids total de la composition.

Ces compositions peuvent se présenter sous forme de dispersions aqueuses, de lotions huileuses, oléoalcooliques, alcooliques ou hydroalcooliques, épaissies ou non et être éventuellement conditionnées en aérosol.

Elles peuvent contenir en plus du diorganopolysiloxane renfermant par molécule au moins un motif de formule (I), des adjuvants habituellement utilisés en cosmétique, tels que des parfums, des colorants, des huiles, des conservateurs, des épaississants, des tensio-actifs, des séquestrants, des stabilisateurs de mousses, des humectants, des filtres solaires, ainsi que des substances actives sur le plan cosmétique.

Les compositions cosmétiques destinées au traitement des cheveux, conformes à l'invention, peuvent être utilisées en particulier comme shampooings, produits à rincer à appliquer avant ou après shampooing, avant ou après coloration ou décoloration, avant ou après permanente ou défrisage, comme produits coiffants non rincés, tels que dans des lotions de mise en plis ou de brushing ou enfin dans des laques.

Lorsque la composition constitue un shampooing, elle contient dans un milieu cosmétiquement acceptable, au moins un ou plusieurs agents de surface anioniques, non ioniques, amphotères ou leurs mélanges; la concentration totale en agents de surface étant généralement comprise entre 0,5 et 30% en poids par rapport au poids total de la composition et de préférence entre 1,5 et 15% en poids.

Lorsque les compositions sont utilisées comme produits à rincer, ces produits peuvent se présenter sous forme de dispersions aqueuses ou de lotions, éventuellement épaissies ou de gels.

Les lotions peuvent contenir dans un milieu aqueux de 1 à 70 % d'un solvant cosmétiquement acceptable, et plus particulièrement choisi parmi les monoalcools inférieurs en $C_1$-$C_4$, tels que l'alcool éthylique, l'alcool isopropylique, l'alcool tertiobutylique, les polyalcools, tels que l'éthylèneglycol, le diéthylèneglycol, le propylèneglycol, les éthers de glycol, tels que les mono- ou diéthylèneglycolalkyléthers, les esters d'acides gras, tels que le myristate d'isopropyle.

Lorsque les compositions sont épaissies ou se présentent sous forme de gel, elles contiennent un ou plusieurs épaississants pouvant être choisis de préférence parmi l'alginate de sodium ou la gomme arabique, les dérivés cellulosiques, tels que la méthylcellulose, l'hydroxyméthylcellulose, l'hydroxyéthylcellulose, l'hydroxypropylcellulose, l'hydroxypropylméthylcellulose, la carboxyméthylcellulose, la gomme de guar ou ses dérivés, la gomme de xanthane ou les scléroglucanes et les polymères d'acide acrylique réticulés ou non. On peut également obtenir un épaississement des compositions par mélange de polyéthylèneglycol et de stéarate ou de distéarate de polyéthylèneglycol ou par un mélange d'esters phosphoriques et d'amides.

On peut épaissir la composition en utilisant le produit résultant de l'interaction ionique d'un polymère cationique constitué par un copolymère de cellulose ou d'un dérivé de cellulose greffé par un sel de monomère hydrosoluble d'ammonium quaternaire et d'un polymère anionique carboxylique ayant une viscosité capillaire absolue dans le diméthylformamide ou le méthanol, à une concentration de 5% et à 30° C, inférieure ou égale à $30 \times 10^{-3}$ Pa.s, tel que décrit plus particulièrement dans la demande de brevet français n° 2 598 611.

Ces épaississants sont utilisés dans des concentrations comprises entre 0,1 et 30% en poids et de

préférence entre 0,2 et 15% en poids par rapport au poids total de la composition.

Lorsque les compositions destinées au traitement des cheveux sont utilisées comme produits coiffants ou non rincés, elles contiennent dans un milieu aqueux ou solvant les diorganopolysiloxanes renfermant par molécule au moins un motif de formule (I) en présence éventuellement d'épaississants tels que définis ci-dessus.

Les solvants sont choisis de préférence parmi les alcools inférieurs en $C_2$-$C_4$ et de préférence ils sont constitués par l'éthanol et les silicones volatiles, telles que les silicones cycliques connues dans le dictionnaire CTFA sous les dénominations HEXAMETHYLDISILOXANE et CYCLOMETHICONE, et leurs mélanges.

Les solvants sont utilisés dans des proportions comprises entre 5% et 99,8% en poids par rapport au poids total de la composition.

Les épaississants particulièrement préférés dans ce cas sont choisis parmi les polymères d'acide acrylique réticulés ou non, et plus particulièrement les acides polyacryliques réticulés par un agent polyfonctionnel tels que les produits vendus sous la dénomination CARBOPOL par la Société GOODRICH, les dérivés de cellulose tels qu'indiqués ci-dessus, les copolymères éthylène anhydride maléique tels que ceux vendus par la Société MONSANTO sous la dénomination EMA 91 et les copolymères de méthylvinyléther et d'anhydride maléique tels que ceux vendus par la Société GAF sous la dénomination GANTREZ AN (119, 139, 169).

La concentration en agents épaississants dans ces compositions varie entre 0,05 et 5% en poids et de préférence entre 0,1 et 2% en poids par rapport au poids total de la composition.

Les compositions conformes à l'invention peuvent être conditionnées en aérosol pour être distribuées sous forme de spray et former des laques. On utilise dans ce cas la composition en présence d'un gaz propulseur tel que plus particulièrement le gaz carbonique, l'azote, le protoxyde d'azote, l'éther diméthylique, les hydrocarbures volatils comme le butane, l'isobutane, le propane, les hydrocarbures chlorés et/ou fluorés et les mélanges d'hydrocarbures tels que le n-butane, l'isobutane, le propane, avec des hydrocarbures chlorofluorés.

Les compositions cosmétiques destinées au traitement et aux soins de la peau peuvent être appliquées en particulier sous forme de produits pour le bain ou la douche, d'huiles corporelles, de produits bronzants, de produits pour le rasage, de lotions parfumées.

Ces compositions contiennent, dans un milieu cosmétiquement acceptable, appropriés pour l'application sur la peau et bien connus de l'homme de métier, les diorganopolysiloxanes définis ci-dessus, dans les proportions également définies.

Les compositions comprenant une substance active sur le plan dermatologique, constituent un autre objet de l'invention; elles contiennent dans un milieu physiologiquement acceptable, défini ci-dessus, au moins un polyorganosiloxane à fonction diester de formule I.

Le procédé de traitement cosmétique mettant en oeuvre les diorganopolysiloxanes renfermant par molécule au moins un motif de formule (I) défini ci-dessus, consiste essentiellement à appliquer la composition, soit sur les cheveux suivant l'usage envisagé (shampooing, traitement à rincer, traitement de coiffage sans rinçage), soit sur la peau (bain, douche, etc...).

Les exemples suivants sont destinés à illustrer l'invention sans pour autant présenter un caractère limitatif.

Les exemples de référence ci-après sont destinés à illustrer la préparation d'un certain nombre de composés conformes à l'invention, alors que les exemples de compositions illustrent l'application cosmétique de ces composés.

## EXEMPLE DE REFERENCE 1

Dans un réacteur tricol de 2 litres, muni d'un réfrigérant, d'un agitateur et d'une ampoule de coulée, on charge 907 g, soit 5,74 moles d'itaconate de méthyle et 129 mg d'acide chloroplatinique ($H_2PtCl_6$).

On porte la température à 116 °C, puis on coule pendant 65 minutes 792,5 g (6,89 moles) de $CH_3HSiCl_2$, soit un excès molaire de 20% par rapport à l'itaconate. La réaction étant exothermique, la température se maintient aux environs de 120 °C sans addition supplémentaire de calories. En fin de coulée, la température est de 112 °C. On maintient le mélange réactionnel au reflux durant 1 heure 50 minutes, puis on distille l'excès de $CH_3HSiCl_2$ et on obtient 1274 g d'un adduct liquide dont le point d'ébullition est de 80 °C sous 0,13 KPa. Le rendement pondéral en adduct est de 71%.

L'analyse RMN de l'adduct indique qu'il présente environ 60% molaire de radicaux

$-(CH_2)-CH-(COOCH_3)-CH_2-COOCH_3$
et 40% molaire de motif
$-C(CH_3)-(COOCH_3)-CH_2-COOCH_3$.

## EXEMPLE DE REFERENCE 2

Préparation d'un composé de formule (II), dans laquelle :
l'un des radicaux R désigne $CH_3$, les deux radicaux terminaux désignent OH;
Z, égal à Z′, est un mélange de radicaux
$-CH_2-CH-(COOCH_3)-CH_2-COOCH_3$
et
$-C(CH_3)-(COOCH_3)-CH_2-COOCH_3$;
r vaut 0;
s représente une valeur statistique moyenne égale à 6,6.

Dans le même réacteur tricol que celui utilisé à l'exemple de référence 1, on charge 340 g d'une solution aqueuse d'ammoniaque ($NH_4OH$ à 20% en poids) et 370 ml d'eau. On coule pendant 50 minutes 500 g (1,83 moles) de l'adduct obtenu à l'exemple 1 en solution dans 500 ml d'éther isopropylique, la température étant maintenue à 25°C.

En fin d'hydrolyse, on décante les eaux mères, puis on ajoute de nouveau 500 ml d'éther isopropylique pour favoriser la décantation. On effectue de nouveau un lavage à l'eau de la solution organique décantée, on sèche et on dévolatilise jusqu'à une température de 100°C sous un vide de 2 KPa.

On obtient alors 323 g d'une huile limpide ayant une teneur pondérale en groupe hydroxyle de 1,8%, une viscosité à 25°C de 35 mPa.s et un pourcentage pondéral en fonction ester de 54,2%.

## EXEMPLE DE REFERENCE 3

Préparation d'un composé de formule (II), dans laquelle :
R désigne $CH_3$;
Z′ désigne OH;
Z est un mélange de radicaux :
$-CH_2-CH-(COOCH_3)-CH_2-COOCH_3$
et
$-C(CH_3)-(COOCH_3)-CH_2-COOCH_3$;
r représente une valeur statistique moyenne égale à 40;
s représente une valeur statistique moyenne égale à 2.

Dans un réacteur tricol de 10 litres, on charge 2800 g d'eau sur laquelle on coule pendant une heure 903 g (7 moles) de $(CH_3)_2SiCl_2$ et 98 g (0,35 mole) de l'adduct obtenu à l'exemple 1. Durant la coulée, la température s'élève graduellement de 25 à 65°C. Après la coulée, on maintient le mélange réactionnel sous agitation pendant 30 minutes et on décante les eaux acides. On ajoute 350 ml d'éther isopropylique, on effectue trois lavages et on concentre la solution éthérée suivant un premier palier jusqu'à 100°C à pression atmosphérique, puis jusqu'à 80°C sous un vide de 2,5 KPa.

On obtient alors 456 g d'huile limpide et incolore, ayant les caractéristiques suivantes :

| | |
|---|---|
| Viscosité à 25°C | 20 mPa.s |
| % (pondéral) d'hydroxyle | 1% |
| % (pondéral) de fonction ester | 4,8% |
| Rendement pondéral en huile | 77% |

## EXEMPLE DE REFERENCE 4

Préparation d'un composé de formule (II), dans laquelle :

Z′ est identique à R et désigne $CH_3$;

Z est un mélange de radicaux :

$-CH_2-CH-(COOCH_3)-CH_2-COOCH_3$

et

$-C(CH_3)-(COOCH_3)-CH_2-COOCH_3$

r vaut 31;

s vaut 17.

On charge dans un réacteur tricol de 5 litres 816 g d'une huile de formule :

$(CH_3)_3SiO(CH_3HSiO)_{17}[(CH_3)_2(SiO)]_{31}Si(CH_3)_3$

et 948 g d'itaconate de méthyle, le tout dans 1540 g de xylène, ainsi que de l'acide chloroplatinique en quantité telle que l'on ait environ 150 ppm de platin-métal par rapport au poids de polymère siloxane. On porte la température à 145° C que l'on maintient pendant 24 heures. Ensuite, on distille le xylène et l'itaconate de méthyle en excès par chauffage à 140° C sous un vide de 2,5 KPa.

On obtient alors 1370 g d'une huile limpide et incolore de viscosité 950 mPa.s à 25° C et un pourcentage pondéral en fonction ester de 28,2%.

## EXEMPLE DE REFERENCE 5

Préparation d'un composé de formule (III).

Dans un réacteur de 1 litre équipé d'un réfrigérant et d'une agitation, on charge en même temps :

- 174 g, soit 1,1 mole d'itaconate de méthyle;
- 46 g d'un hydrogénométhylsiloxane cyclique de pureté 92%, de formule :

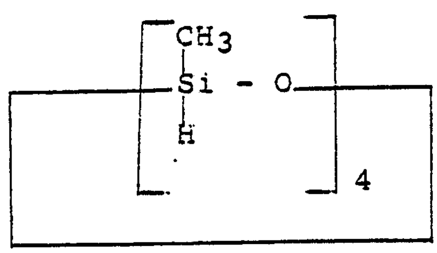

- 25 mg de $H_2PtCl_6(6H_2O)$.

On porte le mélange réactionnel à 135° C et on maintient la température entre 135 et 160° C pendant 1 heure 30 minutes.

On distille l'excès d'itaconate de méthyle du mélange réactionnel à 160° C sous 0,133 KPa. On obtient 146 g d'huile dévolatilisée, de couleur jaune orangé, de viscosité 2240 mPa.s à 25° C, dont le pourcentage pondéral d'ester est de 50%.

Le spectre de masse et le spectre IR ($CHCl_3$) confirment que l'huile obtenue est bien le produit :

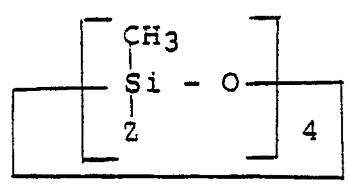

Z étant un radical itaconyle.

## EXEMPLE 1

On prépare un spray de composition suivante :

| | |
|---|---|
| - Diorganopolysiloxane à fonction itaconate de diméthyle de l'exemple de référence 3 | 4 g |
| - Parfum | qs |
| - Alcool éthylique absolu | qsp 100 g |

On conditionne la composition en flacon pompe. Appliqué sur les cheveux séchés, ce spray leur apporte du brillant. Il restitue notamment l'éclat aux cheveux ternis par les traitements chimiques de décoloration ou d'ondulation permanente ou par les agressions atmosphériques, sans les graisser.

## EXEMPLE 2

On prépare un spray de composition suivante :

| | |
|---|---|
| - Diorganopolysiloxane à fonction itaconate de diméthyle de l'exemple de référence 4 | 3 g |
| - Parfum | qs |
| - Alcool éthylique absolu | qsp 100 g |

On conditionne la composition en flacon pompe. Ce spray, comme à l'exemple 1, rend les cheveux brillants sans les graisser.

## EXEMPLE 3

On prépare une composition de soin pour la chevelure à appliquer sur des cheveux séchés :

| | |
|---|---|
| - Diorganopolysiloxane à fonction itaconate de diméthyle de l'exemple de référence 3 | 50 g |
| - Polydiméthylsiloxane PM 500.000 hydroxylé en bout de chaîne en solution à 13% dans un polydiméthylsiloxane cyclique (87%), vendu sous la dénomination Q2 1401 par la Société DOW CORNING | 50 g |

Ce produit de soin rend les cheveux doux, brillants et lisses.

## EXEMPLE 4

On prépare une composition de soin à appliquer sur des cheveux séchés :

| | |
|---|---|
| - Diorganopolysiloxane à fonction itaconate de diméthyle de l'exemple de référence 4 | 50 g |
| - p-méthoxycinnamate de 2-éthylhexyle (PARSOL MCX) | 1 g |
| - Diméthyl p-aminobenzoate de 2-éthylhexyle (PADIMATE O) | 1 g |
| - Diméthylpolysiloxane cyclique, vendu sous l'appellation VOLATILE SILICONE 7158 par la Société UNION CARBIDE | 15 g |
| - Diméthylpolysiloxane cyclique, vendu sous l'appellation VOLATILE SILICONE 7207 par la Société UNION CARBIDE | 15 g |
| - Parfum, conservateur | qs |
| - Alcool éthylique absolu | qsp 100 g |

Cette composition d'application très aisée rend les cheveux doux, souples, brillants, sans leur communiquer un toucher gras.

EXEMPLE 5

On prépare un gel coiffant de composition suivante :

| | |
|---|---|
| - Diorganopolysiloxane à fonction itaconate de diméthyle de l'exemple de référence 3 | 20 g |
| - Acide polyacrylique réticulé PM : 4.000.000, vendu sous la dénomination CARBOPOL 940 par la Société GOODRICH, neutralisé par $NH_4OH$ | 1 g |
| - Parfum | qs |
| - Eau | qsp 100 g |

Appliqué sur cheveux secs, ce gel facilite le maintien de la coiffure et améliore la douceur et le brillant sans communiquer de toucher gras.

EXEMPLE 6

On prépare un shampooing de composition suivante :

| | |
|---|---|
| - Diorganopolysiloxane à fonction itaconate de diméthyle de l'exemple de référence 2 | 5 g |
| - Tensio-actif amphotère dénommé COCOAMPHOCARBOXYGLYCINATE dans le Dictionnaire CTFA (3ème édition), 1982, vendu par la Société MIRANOL à la concentration de 38% de matière active (MA) sous la dénomination de MIRANOL $C_2M$ | 7 g MA |
| - Acide carboxylique d'éther polyglycolique de formule : $R(OCH_2CH_2)_4OCH_2COOH$ où R = mélange de radicaux $C_{12}H_{25}$ et $C_{14}H_{29}$, vendu à 90% de matière active (MA) sous la dénomination AKYPO RLM 45 par la Société CHEM-Y | 2,5 g MA |
| - Polydiméthylsiloxane oxyéthyléné à 70% d'oxyde d'éthylène, vendu sous la dénomination DOW CORNING 193 par la Société DOW CORNING | 3,5 g |
| - HCl qs pH = 4,5 | |
| - Eau | 100 g |

Lavés avec ce shampooing, les cheveux sont doux, souples et sans toucher gras.

## EXEMPLE 7

On prépare un shampooing de composition suivante :

| | |
|---|---|
| - Diorganopolysiloxane à fonction itaconate de diméthyle de l'exemple de référence 3 | 0,4 g |
| - Hydroxyde d'alcoyl $C_{12}$-$C_{18}$ diméthyl carboxyméthyl ammonium, vendu à 30% de matière active (MA) sous la dénomination DEHYTON AB 30 par la Société HENKEL | 10 g MA |
| - Conservateur, parfum | qs |
| - Eau | qsp 100 g |

Ce shampooing aux bonnes propriétés moussantes confère aux cheveux un démêlage facile, du brillant et de la douceur.

EXEMPLE 8

On prépare une huile à appliquer par exemple après le bain, de composition suivante :

| | |
|---|---|
| - Diorganopolysiloxane à fonction itaconate de diméthyle de l'exemple de référence 3 | 40 g |
| - Huile de Colza | 13,3 g |
| - Huile de vaseline | 6,7 g |
| - Décaméthylcyclopentasiloxane | qsp 100,0 g |

Cette huile confère à la peau souplesse et douceur.

**Revendications**

1. Utilisation en cosmétique ou dermatologie d'un diorganopolysiloxane à fonction diester, portant, par molécule, au moins un motif de formule :

$$ZR_aSiO_{\frac{3-a}{2}} \qquad (I)$$

dans laquelle :
- a est 0, 1 ou 2
- Z désigne un radical de formule :

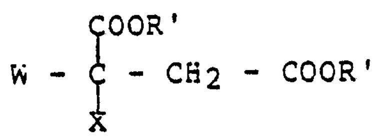

dans laquelle :
les symboles R′, identiques ou différents, désignent un radical hydrocarboné saturé, monovalent en $C_1$-$C_{12}$, un radical alcoxyalkyle monovalent en $C_2$-$C_{12}$, un radical aryle, alkylaryle ou aralkyle en $C_6$-$C_{12}$;
X désigne un atome d'hydrogène, un radical méthyle;
W désigne une liaison covalente ou un radical alkylène, linéaire ou ramifié en $C_1$-$C_4$;
les symboles R, identiques ou différents, désignent un radical alkyle en $C_1$-$C_{20}$, vinyle, phényle, trifluoro-3,3,3 propyle ou hydroxyle, sous réserve qu'un seul radical R par atome de silicium puisse être un groupement hydroxyle; ainsi que ses mélanges.

2. Utilisation selon la revendication 1, caractérisée par le fait que le diorganopolysiloxane à fonction diester porte également d'autres motifs siloxyle, répondant à la formule :

$$R_bSiO_{\frac{4-b}{2}} \qquad (Ibis)$$

dans laquelle :
R a les mêmes significations que ci-dessus et b est égal e à 0, 1, 2 ou 3.

3. Utilisation selon la revendication 1 ou 2, caractérisée par le fait que dans les diorganopolysiloxanes portant par molécule au moins un motif de formule (I), les groupements alkyle en $C_1$-$C_{12}$ représentés par R′ sont choisis parmi les radicaux méthyle, éthyle, n-propyle, isopropyle, n-butyle, éthyl-2 hexyle, heptyle et docécyle; les radicaux aryle, aralkyle et alkylaryle en $C_6$-$C_{12}$ sont choisis parmi le phényle, le benzyle et le tolyle; les radicaux alcoxyalkyle en $C_2$-$C_{12}$ sont choisis parmi les radicaux méthoxyméthyle, éthoxyméthyle et méthoxy-2 éthyle; le radical W désigne des radicaux alkylène choisis parmi :

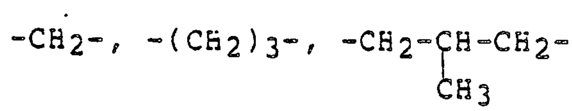

lorsque X désigne un hydrogène ou encore W est une liaison covalente lorsque X est un groupement méthyle;

R désigne un radical méthyle, éthyle, propyle, n-butyle, n-octyle ou éthyl-2 hexyle.

4. Utilisation selon l'une quelconque des revendications 1 à 3, caractérisée par le fait que dans les diorganopolysiloxanes portant par molécule au moins un motif de formule (I), 80% en nombre des radicaux R sont des méthyle.

5. Utilisation en cosmétique ou dermatologie de diorganopolysiloxanes selon l'une quelconque des revendications 1 à 4, caractérisée par le fait qu'ils sont choisis parmi :

(i) les composés répondant à la formula :

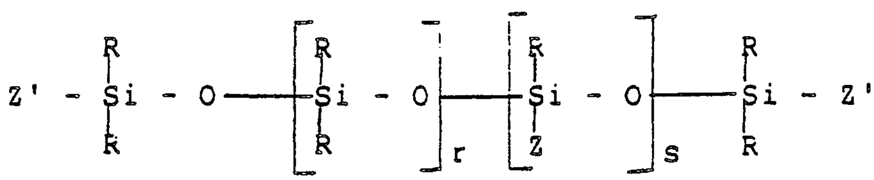

(II)

dans laquelle :

R a la signification définie dans la revendication 1;

les symboles Z′, identiques ou différents, sont choisis parmi Z ou R;

Z est choisi parmi les radicaux

- $CH_2$-CH-(COOR′)$CH_2$COOR′

- C($CH_3$)(COOR′)$CH_2$COOR′

R′ ayant la signification définie dans la revendication 1;

r est un nombre entier compris entre 0 et 500 inclusivement;

s est un nombre entier choisi entre 0 et 50 inclusivement; et

si s est égal à 0, un des symboles Z′ désigne Z;

(ii) les polysiloxanes cycliques de formule :

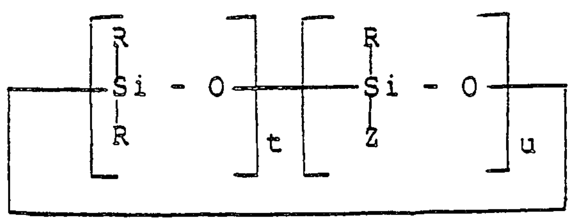

(III)

dans laquelle :

R et Z ont la signification définie ci-dessus;

u est un nombre entier compris entre 1 et 20 inclus;

t est un nombre entier compris entre 0 et 20 inclus;

t + u est supérieur ou égal à 3.

6. Utilisation en cosmétique ou dermatologie selon l'une quelconque des revendications 1 à 5, caractérisée par le fait que les diorganopolysiloxanes sont des polymères statistiques ou à blocs, présentant l'une des caractéristiques suivantes :

a) R et R′ sont méthyle

b) r est compris entre 5 et 50 inclus

c) s est compris entre 1 et 20 inclus

d) t + u est compris entre 3 et 10 inclus.

7. Composition de traitement cosmétique des cheveux ou de la peau, caractérisée par le fait qu'elle contient dans un milieu cosmétiquement acceptable au moins un diorganopolysiloxane à fonction diester portant par molécule au moins un motif de formule (I), tel que défini dans l'une quelconque des revendications 1 à 6, dans des concentrations comprises entre 0,2 et 90%.

8. Composition selon la revendication 7, caractérisée par le fait qu'elle contient dans un milieu cosmétiquement acceptable, un diorganopolysiloxane à fonction diester, tel que défini dans l'une quelconque des revendications 1 à 6, dans des concentrations comprises entre 0,3 et 60% en poids par rapport au poids total de la composition.

9. Composition selon la revendication 7 ou 8, caractérisée par le fait qu'elle se présente sous forme de dispersions aqueuses, de lotions huileuses, oléoalcooliques, alcooliques ou hydroalcooliques, épaissies ou non, éventuellement conditionnées en aérosol.

10. Composition selon l'une des revendications 7 à 9, caractérisée par le fait qu'elle contient au moins un ou plusieurs adjuvants habituellement utilisés en cosmétique, choisis parmi les parfums, les colorants, les huiles, les conservateurs, les épaississants, les agents tensio-actifs anioniques, non ioniques ou amphotères ou leurs mélanges, les séquestrants, les stabilisateurs de mousses, les humectants, les filtres solaires, les substances actives en cosmétique et en dermatologie.

11. Composition selon l'une quelconque des revendications 7 à 10, destinée à être utilisée comme shampooing, caractérisée par le fait qu'elle contient en plus au moins un ou plusieurs agents de surface anioniques, non ioniques, amphotères ou leurs mélanges, dans des proportions comprises entre 0,5 et 30% en poids par rapport au poids total de la composition.

12. Composition selon l'une quelconque des revendications 7 à 11, destinée à être utilisée comme produit à rincer, caractérisée par le fait qu'elle se présente sous forme de dispersion aqueuse, de lotion, de lotion épaissie ou de gel.

13. Composition selon la revendication 12, caractérisée par le fait qu'elle contient au moins 1 à 70% de solvant choisi parmi les alcanols inférieurs, les polyalcools, les éthers de glycol et les esters d'acides gras.

14. Composition selon l'une quelconque des revendications 7 à 13, caractérisée par le fait qu'elle est épaissie ou gélifiée avec un agent choisi parmi l'alginate de sodium, la gomme arabique, les dérivés cellulosiques, la gomme de guar, la gomme de xanthane et les shléroglucanes, les polymères d'acide acrylique réticulés ou non, un mélange de polyéthylèneglycol et de stéarate ou de distéarate de polyéthylèneglycol, un mélange d'ester phosphorique et d'amide, le produit d'interaction ionique entre un polymère cationique constitué par un copolymère de cellulose ou un dérivé de cellulose greffé par un sel de monomère hydrosoluble d'ammonium quaternaire et un polymère anionique carboxylique ayant une viscosité capillaire absolue, dans le diméthylformamide ou le méthanol, à une concentration de 5% et à 30°C, inférieure ou égale à $30 \times 10^{-3}$ Pa.s, cet agent épaississant est présent dans des proportions comprises entre 0,1 et 30% en poids par rapport au poids total de la composition.

15. Composition selon l'une quelconque des revendications 7 à 10, destinée à être utilisée comme produits coiffants, sans mettre en oeuvre de rinçage, caractérisée par le fait qu'elle contient en milieu aqueux ou solvant, les diorganopolysiloxanes à fonction diester tels que définis dans les revendications 1 à 6, les solvants étant choisis parmi les alcanols inférieurs en $C_2$-$C_4$ et les silicones volatiles.

16. Composition selon la revendication 15, caractérisée par le fait qu'elle contient les épaississants choisis parmi les polymères d'acide acrylique réticulés ou non, les dérivés cellulosiques, les copolymères éthylène anhydride maléique, les copolymères de méthylvinyléther et d'anhydride maléique, dans des proportions comprises entre 0,05 et 5% en poids.

17. Composition selon l'une quelconque des revendications 7 à 16, caractérisée par le fait qu'elle est conditionnée en aérosol pour former au moment de l'expulsion un spray, en présence d'un gaz propulseur.

18. Utilisation de la composition telle que définie dans l'une quelconque des revendications 7 à 17, dans le traitement cosmétique des cheveux.

19. Utilisation de la composition selon l'une quelconque des revendications 7 à 10, comme produits de bain ou de douche, d'huiles corporelles, de produits bronzants, de produits pour le rasage, de lotions parfumées.

20. Procédé de traitement cosmétique des cheveux, caractérisé par le fait que l'on applique sur ceux-ci au moins une composition telle que définie dans l'une quelconque des revendications 7 à 17.

21. Procédé de traitement cosmétique de la peau, caractérisé par le fait que l'on applique sur celle-ci au moins une composition telle que définie dans l'une quelconque des revendications 7 à 10.

22. Composition dermatologique comprenant une substance active dermatologique, caractérisée par le fait qu'elle contient dans un milieu physiologiquement acceptable, au moins un diorganopolysiloxane à fonction diester tel que défini dans les revendications 1 et 6.

Revendications pour l'Etat contractant suivant : ES

1. Procédé de préparation d'une composition cosmétique pour le traitement des cheveux ou de la peau, sous forme de dispersion aqueuse, de lotion alcoolique, oléoalcoolique, huileuse ou hydroalcoolique,

épaissie ou non, éventuellement conditionnée en aérosol, caractérisé par le fait que l'on incorpore dans un milieu cosmétiquement acceptable, dans des proportions comprises entre 0,2 et 90% et de préférence entre 0,3 et 60% en poids par rapport au poids total de la composition, au moins un diorganopolysiloxane à fonction diester, portant, par molécule, au moins un motif de formule :

$$ZR_aSiO_{\frac{3-a}{2}} \qquad (I)$$

dans laquelle :
- a est 0, 1 ou 2
- Z désigne un radical de formule :

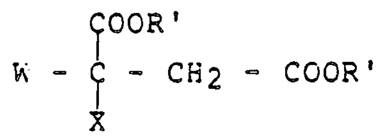

dans laquelle :
les symboles R', identiques ou différents, désignent un radical hydrocarboné saturé, monovalent en $C_1$-$C_{12}$, un radical alcoxyalkyle monovalent en $C_2$-$C_{12}$, un radical aryle, alkylaryle ou aralkyle en $C_6$-$C_{12}$;
X désigne un atome d'hydrogène, un radical méthyle;
W désigne une liaison covalente ou un radical alkylène, linéaire ou ramifié en $C_1$-$C_4$;
les symboles R, identiques ou différents, désignent un radical alkyle en $C_1$-$C_{20}$, vinyle, phényle, trifluoro-3,3,3 propyle ou hydroxyle, sous réserve qu'un seul radical R par atome de silicium puisse être un groupement hydroxyle; ainsi que les mélanges.

2. Procédé selon la revendication 1, caractérisé par le fait que le diorganopolysiloxane à fonction diester porte également d'autres motifs siloxyle, répondant à la formule :

$$R_bSiO_{\frac{4-b}{2}} \qquad (Ibis)$$

dans laquelle :
R a les mêmes significations que ci-dessus et b est égal e à 0, 1, 2 ou 3.

3. Procédé selon la revendication 1 ou 2, caractérisé par le fait que dans les diorganopolysiloxanes portant par molécule au moins un motif de formule (I), les groupements alkyle en $C_1$-$C_{12}$ représentés par R' sont choisis parmi les radicaux méthyle, éthyle, n-propyle, isopropyle, n-butyle, éthyl-2 hexyle, heptyle et docécyle; les radicaux aryle, aralkyle et alkylaryle en $C_6$-$C_{12}$ sont choisis parmi le phényle, le benzyle et le tolyle; les radicaux alcoxyalkyle en $C_2$-$C_{12}$ sont choisis parmi les radicaux méthoxyméthyle, éthoxyméthyle et méthoxy-2 éthyle; le radical W désigne des radicaux alkylène choisis parmi :

$-CH_2-$, $-(CH_2)_3-$, 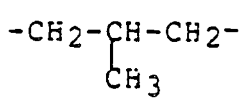

lorsque X désigne un hydrogène ou encore W est une liaison covalente lorsque X est un groupement méthyle;
R désigne un radical méthyle, éthyle, propyle, n-butyle, n-octyle ou éthyl-2 hexyle.

4. Procédé selon l'une quelconque des revendications 1 à 3, caractérisé par le fait que dans les diorganopolysiloxanes portant par molécule au moins un motif de formule (I), 80% en nombre des radicaux R sont des méthyle.

5. Procédé selon l'une quelconque des revendications 1 à 4, caractérisé par le fait que les diorganopolysiloxanes sont choisis parmi :

(i) les composés répondant à la formula :

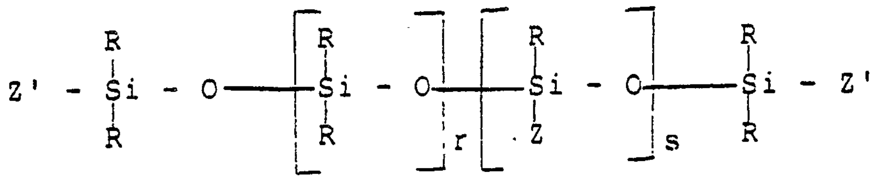 (II)

dans laquelle :
R a la signification définie dans la revendication 1;
les symboles Z′, identiques ou différents, sont choisis parmi Z ou R;
Z est choisi parmi les radicaux
- $CH_2$-CH-(COOR′)$CH_2$COOR′
- C($CH_3$)(COOR′)$CH_2$COOR′
R′ ayant la signification définie dans la revendication 1;
r est un nombre entier compris entre 0 et 500 inclusivement;
s est un nombre entier choisi entre 0 et 50 inclusivement; et
si s est égal à 0, un des symboles Z′ désigne Z;
(ii) les polysiloxanes cycliques de formule :

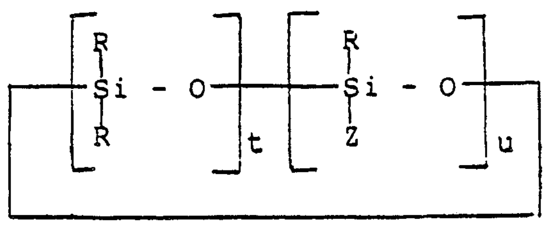 (III)

dans laquelle :
R et Z ont la signification définie ci-dessus;
u est un nombre entier compris entre 1 et 20 inclus;
t est un nombre entier compris entre 0 et 20 inclus;
t + u est supérieur ou égal à 3.

6. Procédé selon l'une quelconque des revendications 1 à 5, caractérisé par le fait que les diorganopolysiloxanes sont des polymères statistiques ou à blocs, présentant l'une des caractéristiques suivantes :
a) R et R′ sont méthyle
b) r est compris entre 5 et 50 inclus
c) s est compris entre 1 et 20 inclus
d) t+u est compris entre 3 et 10 inclus.

7. Procédé selon l'une des revendications 1 à 6, caractérisé par le fait que l'on ajoute au moins un ou plusieurs adjuvants habituellement utilisés en cosmétique, choisis parmi les parfums, les colorants, les huiles, les conservateurs, les épaississants, les agents tensio-actifs anioniques, non ioniques ou amphotères ou leurs mélanges, les séquestrants, les stabilisateurs de mousses, les humectants, les filtres solaires, les substances actives en cosmétique.

8. Procédé de préparation d'une composition selon l'une quelconque des revendications 1 à 7, destinée à être utilisée comme shampooing, caractérisé par le fait que l'on ajoute en plus au moins un ou plusieurs agents de surface anioniques, non ioniques, amphotères ou leurs mélanges, dans des proportions comprises entre 0,5 et 30% en poids par rapport au poids total de la composition.

9. Procédé de préparation d'une composition selon l'une quelconque des revendications 1 à 8, destinée à être utilisée comme produit à rincer, caractérisé par le fait que l'on prépare une composition sous forme de dispersion aqueuse, de lotion, de lotion épaissie ou de gel.

10. Procédé de préparation d'une composition selon la revendication 9, caractérisé par le fait qu'elle contient au moins 1 à 70% de solvant choisi parmi les alcanols inférieurs, les polyalcools, les éthers de glycol et les esters d'acides gras.

11. Procédé de préparation d'une composition selon l'une quelconque des revendications 1 à 10, caractérisé par le fait qu'elle est épaissie ou gélifiée avec un agent choisi parmi l'alginate de sodium, la gomme arabique, les dérivés cellulosiques, la gomme de guar, la gomme de xanthane et les scléroglucanes, les polymères d'acide acrylique réticulés ou non, un mélange de polyéthylèneglycol et de stéarate ou

de distéarate de polyéthylèneglycol, un mélange d'ester phosphorique et d'amide, le produit d'interaction ionique entre un polymère cationique constitué par un copolymère de cellulose ou un dérivé de cellulose greffé par un sel de monomère hydrosoluble d'ammonium quaternaire et un polymère anionique carboxylique ayant une viscosité capillaire absolue, dans le diméthylformamide ou le méthanol, à une concentration de 5% et à 30 °C, inférieure ou égale à $30 \times 10^{-3}$ Pa.s, cet agent épaississant est présent dans des proportions comprises entre 0,1 et 30% en poids par rapport au poids total de la composition.

12. Procédé de préparation d'une composition selon l'une quelconque des revendications 1 à 7, destinée à être utilisée comme produits coiffants, sans mettre en oeuvre de rinçage, caractérisé par le fait qu'elle contient en milieu aqueux ou solvant, les diorganopolysiloxanes à fonction diester tels que définis dans les revendications 1 à 6, les solvants étant choisis parmi les alcanols inférieurs en $C_2$-$C_4$ et les silicones volatiles.

13. Procédé de préparation d'une composition selon la revendication 12, caractérisé par le fait qu'elle contient les épaississants choisis parmi les polymères d'acide acrylique réticulés ou non, les dérivés cellulosiques, les copolymères éthylène anhydride maléique, les copolymères de méthylvinyléther et d'anhydride maléique, dans des proportions comprises entre 0,05 et 5% en poids.

14. Procédé de préparation d'une composition selon l'une quelconque des revendications 1 à 13, caractérisé par le fait qu'elle est conditionnée en aérosol pour former au moment de l'expulsion un spray, en présence d'un gaz propulseur.

15. Procédé de préparation d'une composition dermatologique, caractérisé par le fait que l'on incorpore dans un milieu physiologiquement acceptable, au moins un substance active dermatologique dans une quantité efficace et au moins un diorganopolysiloxane à fonction diester, tel que défini dans l'une quelconque des revendications 1 à 6, dans des proportions comprises entre 0,2 et 90% et de préférence entre 0,3 et 60% en poids par rapport au poids total de la composition.

16. Composition cosmétique pour le traitement des cheveux ou de la peau sous forme de dispersion aqeuse, de lotion alcoolique, huileuse, oléoalcoolique ou hydroalcoolique, épaissie ou non, éventuellement conditionnée en aérosol, caractérisée par le fait qu'elle contient dans un milieu cosmétiquement acceptable, dans des proportions comprises entre 0,2 et 90% et de préférence entre 0,3 et 60% en poids par rapport au poids total de la composition, au moins un diorganopolysiloxane à fonction diester, tel que défini dans l'une quelconque des revendications 1 à 6.

Revendications pour l'Etat contractant suivant : GR

1. Utilisation en cosmétique d'un diorganopolysiloxane à fonction diester, portant, par molécule, au moins un motif de formule :

$$ZR_aSiO_{\frac{3-a}{2}} \qquad (I)$$

dans laquelle :
- a est 0, 1 ou 2
- Z désigne un radical de formule :

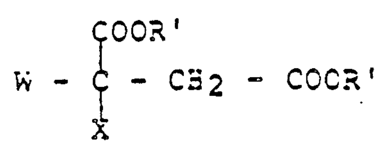

dans laquelle :
les symboles R′, identiques ou différents, désignent un radical hydrocarboné saturé, monovalent en $C_1$-$C_{12}$, un radical alcoxyalkyle monovalent en $C_2$-$C_{12}$, un radical aryle, alkylaryle ou aralkyle en $C_6$-$C_{12}$;
X désigne un atome d'hydrogène, un radical méthyle;
W désigne une liaison covalente ou un radical alkylène, linéaire ou ramifié en $C_1$-$C_4$;
les symboles R, identiques ou différents, désignent un radical alkyle en $C_1$-$C_{20}$, vinyle, phényle, trifluoro-3,3,3 propyle ou hydroxyle, sous réserve qu'un seul radical R par atome de silicium puisse être un groupement hydroxyle; ainsi que ses mélanges.

2. Utilisation selon la revendication 1, caractérisée par le fait que le diorganopolysiloxane à fonction

diester porte également d'autres motifs siloxyle, répondant à la formule :

$$R_bSiO_{\frac{4-b}{2}} \qquad (Ibis)$$

dans laquelle :

R a les mêmes significations que ci-dessus et b est égal e à 0, 1, 2 ou 3.

3. Utilisation selon la revendication 1 ou 2, caractérisée par le fait que dans les diorganopolysiloxanes portant par molécule au moins un motif de formule (I), les groupements alkyle en $C_1$-$C_{12}$ représentés par R′ sont choisis parmi les radicaux méthyle, éthyle, n-propyle, isopropyle, n-butyle, éthyl-2 hexyle, heptyle et docécyle; les radicaux aryle, aralkyle et alkylaryle en $C_6$-$C_{12}$ sont choisis parmi le phényle, le benzyle et le tolyle; les radicaux alcoxyalkyle en $C_2$-$C_{12}$ sont choisis parmi les radicaux méthoxyméthyle, éthoxyméthyle et méthoxy-2 éthyle; le radical W désigne des radicaux alkylène choisis parmi :

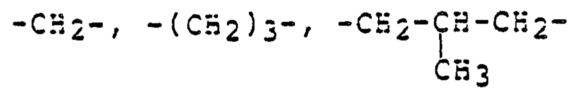

lorsque X désigne un hydrogène ou encore W est une liaison covalente lorsque X est un groupement méthyle;

R désigne un radical méthyle, éthyle, propyle, n-butyle, n-octyle ou éthyl-2 hexyle.

4. Utilisation selon l'une quelconque des revendications 1 à 3, caractérisé par le fait que dans les diorganopolysiloxanes portant par molécule au moins un motif de formule (I), 80% en nombre des radicaux R sont des méthyle.

5. Utilisation en cosmétique de diorganopolysiloxanes selon l'une quelconque des revendications 1 à 4, caractérisée par le fait qu'ils sont choisis parmi :

(i) les composés répondant à la formule :

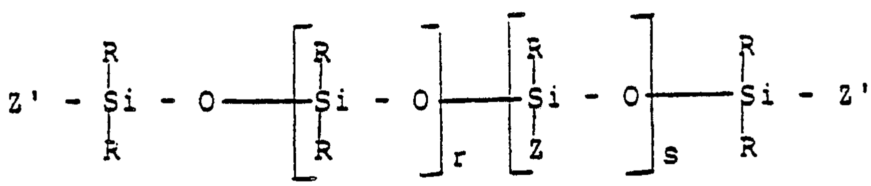

(II)

dans laquelle :

R a la signification définie dans la revendication 1;

les symboles Z′, identiques ou différents, sont choisis parmi Z ou R;

Z est choisi parmi les radicaux

- $CH_2$-CH-(COOR′)$CH_2$COOR′

- C($CH_3$)(COOR′)$CH_2$COOR′

R′ ayant la signification définie dans la revendication 1;

r est un nombre entier compris entre 0 et 500 inclusivement;

s est un nombre entier choisi entre 0 et 50 inclusivement; et

si s est égal à 0, un des symboles Z′ désigne Z;

(ii) les polysiloxanes cycliques de formule :

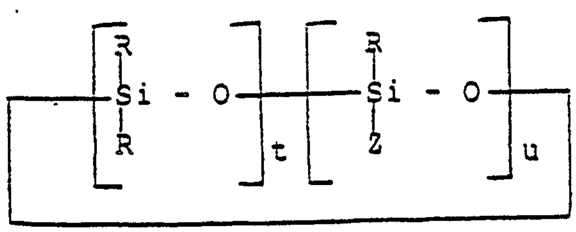

(III)

dans laquelle :
R et Z ont la signification définie ci-dessus;
u est un nombre entier compris entre 1 et 20 inclus;
t est un nombre entier compris entre 0 et 20 inclus;
t + u est supérieur ou égal à 3.

6. Utilisation en cosmétique selon l'une quelconque des revendications 1 à 5, caractérisée par le fait que les diorganopolysiloxanes sont des polymères statistiques ou à blocs, présentant l'une des caractéristiques suivantes :

a) R et R′ sont méthyle
b) r est compris entre 5 et 50 inclus
c) s est compris entre 1 et 20 inclus
d) t+u est compris entre 3 et 10 inclus.

7. Composition de traitement cosmétique des cheveux ou de la peau, caractérisée par le fait qu'elle contient dans un milieu cosmétiquement acceptable au moins un diorganopolysiloxane à fonction diester portant par molécule au moins un motif de formule (I), tel que défini dans l'une quelconque des revendications 1 à 6, dans des concentrations comprises entre 0,2 et 90%.

8. Composition selon la revendication 7, caractérisée par le fait qu'elle contient dans un milieu cosmétiquement acceptable, un diorganopolysiloxane à fonction diester, tel que défini dans l'une quelconque des revendications 1 à 6, dans des concentrations comprises entre 0,3 et 60% en poids par rapport au poids total de la composition.

9. Composition selon la revendication 7 ou 8, caractérisée par le fait qu'elle se présente sous forme de dispersions aqueuses, de lotions huileuses, oléoalcooliques, alcooliques ou hydroalcooliques, épaissies ou non, éventuellement conditionnées en aérosol.

10. Composition selon l'une des revendications 7 à 9, caractérisée par le fait qu'elle contient au moins un ou plusieurs adjuvants habituellement utilisés en cosmétique, choisis parmi les parfums, les colorants, les huiles, les conservateurs, les épaississants, les agents tensio-actifs anioniques, non ioniques ou amphotères ou leurs mélanges, les séquestrants, les stabilisateurs de mousses, les humectants, les filtres solaires, les substances actives en cosmétique.

11. Composition selon l'une quelconque des revendications 7 à 10, destinée à être utilisée comme shampooing, caractérisée par le fait qu'elle contient en plus au moins un ou plusieurs agents de surface anioniques, non ioniques, amphotères ou leurs mélanges, dans des proportions comprises entre 0,5 et 30% en poids par rapport au poids total de la composition.

12. Composition selon l'une quelconque des revendications 7 à 11, destinée à être utilisée comme produit à rincer, caractérisée par le fait qu'elle se présente sous forme de dispersion aqueuse, de lotion, de lotion épaissie ou de gel.

13. Composition selon la revendication 12, caractérisée par le fait qu'elle contient au moins 1 à 70% de solvant choisi parmi les alcanols inférieurs, les polyalcools, les éthers de glycol et les esters d'acides gras.

14. Composition selon l'une quelconque des revendications 7 à 13, caractérisée par le fait qu'elle est épaissie ou gélifiée avec un agent choisi parmi l'alginate de sodium, la gomme arabique, les dérivés cellulosiques, la gomme de guar, la gomme de xanthane et les scléroglucanes, les polymères d'acide acrylique réticulés ou non, un mélange de polyéthylèneglycol et de stéarate ou de distéarate de polyéthylèneglycol, un mélange d'ester phosphorique et d'amide, le produit d'interaction ionique entre un polymère cationique constitué par un copolymère de cellulose ou un dérivé de cellulose greffé par un sel de monomère hydrosoluble d'ammonium quaternaire et un polymère anionique carboxylique ayant une viscosité capillaire absolue, dans le diméthylformamide ou le méthanol, à une concentration de 5% et à 30 °C, inférieure ou égale à $30 \times 10^{-3}$ Pa.s, cet agent épaississant est présent dans des proportions comprises entre 0,1 et 30% en poids par rapport au poids total de la composition.

15. Composition selon l'une quelconque des revendications 7 à 10, destinée à être utilisée comme produits coiffants, sans mettre en oeuvre de rinçage, caractérisée par le fait qu'elle contient en milieu aqueux ou solvant, les diorganopolysiloxanes à fonction diester tels que définis dans les revendications 1 à 6, les solvants étant choisis parmi les alcanols inférieurs en $C_2$-$C_4$ et les silicones volatiles.

16. Composition selon la revendication 15, caractérisée par le fait qu'elle contient les épaississants choisis parmi les polymères d'acide acrylique réticulés ou non, les dérivés cellulosiques, les copolymères éthylène anhydride maléique, les copolymères de méthylvinyléther et d'anhydride maléique, dans des proportions comprises entre 0,05 et 5% en poids.

17. Composition selon l'une quelconque des revendications 7 à 16, caractérisée par le fait qu'elle est conditionnée en aérosol pour former au moment de l'expulsion un spray, en présence d'un gaz propulseur.

18. Utilisation de la composition telle que définie dans l'une quelconque des revendications 7 à 17,

dans le traitement cosmétique des cheveux.

19. Utilisation de la composition selon l'une quelconque des revendications 7 à 10, comme produits de bain ou de douche, d'huiles corporelles, de produits bronzants, de produits pour le rasage, de lotions parfumées.

20. Procédé de traitement cosmétique des cheveux, caractérisé par le fait que l'on applique sur ceux-ci au moins une composition telle que définie dans l'une quelconque des revendications 7 à 17.

21. Procédé de traitement cosmétique de la peau, caractérisé par le fait que l'on applique sur celle-ci au moins une composition telle que définie dans l'une quelconque des revendications 7 à 10.

22. Procédé de préparation d'une composition dermatologique, caractérisé par le fait que l'on introduit dans un milieu physiologiquement acceptable une substance active dermatologique dans une quantité efficace et au moins un diorganopolysiloxane à fonction diester, tel que défini dans les revendications 1 à 6, dans des proportions comprises entre 0,2 et 90% en poids et de préférence entre 0,3 et 60% en poids par rapport au poids total de la composition.

Office européen des brevets

# RAPPORT DE RECHERCHE EUROPEENNE

Numero de la demande

EP 89 40 2628

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,Y | EP-A-0 004 074 (CONSORTIUM FÜR ELEKTROCHEMISCHE INDUSTRIE)<br>* Revendications *<br>--- | 1-22 | A 61 K 7/06<br>A 61 K 7/48<br>C 08 G 77/38 |
| Y | EP-A-0 186 438 (DOW)<br>* Revendications; page 4, ligne 9 - page 8, ligne 13; page 11, paragraphe 4; page 12, paragraphe 1 *<br>----- | 1-22 | |
| | | | **DOMAINES TECHNIQUES RECHERCHES (Int. Cl.5)** |
| | | | A 61 K<br>C 08 G |

Le présent rapport a été établi pour toutes les revendications

| Lieu de la recherche | Date d'achèvement de la recherche | Examinateur |
|---|---|---|
| LA HAYE | 12-12-1989 | WILLEKENS G.E.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)